# EUROPEAN PATENT APPLICATION

(11) **EP 1 736 141 A1**
(43) Date of publication of application: **27.12.2006**
(21) Application number: 06290935.3
(22) Date of filing: 08.06.2006
(51) Int. Cl.: A61K 8/34, A61Q 9/02

(54) **Shaving preparation**

(30) Priority: 23.06.2005 US 159774
(71) Applicant: L'Oreal, 75008 Paris (FR)
(72) Inventor: Fares, Hani, Somerset, NJ 08873 (US); Zambell, Christopher, Jersey City, NJ 07310 (US); Hansenne, Isabelle, Westfield, NJ 07090 (US)
(74) Representative: Miszputen, Laurent

(57) **Abstract**

The present invention is directed to a composition and process for shaving a surface, such as human skin having hair projecting therefrom. The process involves the steps of: (a) providing a surface to be shaved; (b) providing a composition containing: (i) at least one nonaqueous polar organic compound having at least two hydroxyl groups; (ii) optionally, at least one emollient; (iii) optionally, at least one thickening agent; (iv) optionally, at least one fragrance; and (v) optionally, water; (c) optionally, wetting the surface to be shaved; (d) applying the composition onto the surface to be shaved in order to form a treated surface; and (e) shaving the treated surface.

## Description

### BACKGROUND OF THE INVENTION

Shaving preparations have long been available as powders, creams, and foams formulated to exhibit excellent lathering ability. More recently, consumers have been introduced to gel shaving compositions, which can be worked into a lather when rubbed against the skin.

Shaving preparations generally include soap as a major component. As used herein, "soap" means a salt of a fatty acid with a base. Examples are the salts of fatty acids with ammonia, low molecular weight amines (especially alkanolamines), and alkali metals (especially sodium and potassium). Other fatty acid salts result from the reaction of metallic cations (e.g., zinc, aluminum, and alkaline earth metals such as calcium and magnesium) with long chain fatty amines. The most common soaps used in shaving preparations are stearates and palmitates.

A drawback to using soap, however, lies in its tendency to cause irritation to the skin, particularly to delicate and broken skin, during shaving. Soap-containing shaving preparations can also cause drying of the skin and premature dulling of razor blades.

Accordingly, in recent years, the cosmetics industry has developed soap-free, non-lathering shaving preparations. By non-lathering, we mean shaving preparations that do not contain surfactants of the lathering or foaming type, as understood by one of ordinary skill in the art.

While prior art soap-free formulations solved certain problems associated with skin irritation and drying by incorporating moisturizers and skin softeners into their formulations, the presence of such ingredients adversely affected the subsequent rinsability of the shaving preparation, leaving the shaved skin feeling greasy and/or sticky.

### SUMMARY OF THE INVENTION

The present invention is directed to a process for shaving involving:
(a) providing a surface to be shaved;
(b) providing a composition containing:
   (i) at least one non-aqueous polar organic compound having at least two hydroxyl groups;
   (ii) optionally, at least one emollient;
   (iii) optionally, at least one thickening agent;
   (iv) optionally, at least one fragrance; and
   (v) optionally, water;
(c) optionally, wetting the surface to be shaved;
(d) applying the composition onto the surface to be shaved in order to form a treated surface; and
(e) shaving the treated surface.

The present invention is also directed to a shaving composition containing:
(a) at least 50% by weight of at least one non-aqueous polar organic compound having at least two hydroxyl groups;
(b) from 0.01 to 20% by weight of at least one emollient;
(c) from 0.01 to 10% by weight of at least one thickening agent;
(d) from 0.01 to 5% by weight of at least one fragrance; and
(e) optionally, up to 20% by weight, water, all weights being based on the total weight of the composition.

### DETAILED DESCRIPTION

Other than in the operating examples, or where otherwise indicated, all numbers expressing quantities of ingredients and/or reaction conditions are to be understood as being modified in all instances by the term "about".

The most important component of the present invention is the non-aqueous polar organic compound. A variety of non-aqueous polar organic compounds are suitable for use in the present invention. Examples are as follows.

Polyols

Polyols are suitable nonaqueous polar organic compounds. For purposes of this specification, polyols are defined as compounds which contain two or more hydroxyl groups per molecule. Examples of suitable polyols include fructose, glucamine, glucose, glucose glutamate, glucuronic acid, glycerin, 1,2,6hexanetriol, hydroxystearyl methylglucanine, inositol, lactose, malitol, mannitol, methyl gluceth-10, methyl gluceth-20, methyl glucose dioleate, methyl glucose sesquicaprylate/sesquicaprate, methyl glucose sesquicocoate, methyl glucose sesquiisostearate, methyl glucose sesquilaurate, methyl glucose sesquistearate, phytantriol, riboflavin, sorbeth-6, sorbeth-20, sorbeth-30, sorbeth-40, sorbitol, sucrose, thioglycerin, xylitol, and mix thereof. An especially preferred polyol is glycerin.

Polymeric or Monomeric Ethers

Also suitable as the non-aqueous polar organic material are homopolymeric or block copolymeric liquid ethers. Polymeric ethers are preferably formed by polymerization of monomeric alkylene oxides, generally ethylene or propylene oxides. Examples of such polymeric ethers include PEG, PPG, and derivatives thereof.

Other examples of suitable polymeric ethers include polyoxypropylene polyoxyethylene block copolymers. Such compounds are sold under the CTFA name Meroxapol 105, 108, 171, 172, 174, 178, 251, 252, 254, 255, 258, 311, 312, and 314.

Dihydric Alcohols

Also suitable for use as to the non-aqueous polar organic compound are dihydric alcohols of the general formula R(OH)ₙ where n is 2 and R is a substituted or unsubstituted saturated C₂₋₁₀, preferably C₁₋₈ alkyl, or a substituted or unsubstituted alicyclic, bicyclic, or aromatic ring, with the substituents selected from halogen, alkoxy, hydroxy, and so on. Examples of suitable dihydric alcohols include, but are not limited to, hexylene glycol, diethylene glycol, ethylene glycol, propylene glycol, 1,2-butylene glycol, triethylene glycol, dipropylene glycol, and mixtures thereof.

Sorbitan Derivatives

Sorbitan derivatives, which are defined as ethers or esters of sorbitan, are also suitable polar solvents. Examples of suitable sorbitan derivatives are the Polysorbates, which are defined as stearate esters of sorbitol and sorbitan anhydrides, such as Polysorbate 20, 21, 40, 60, 61, 65, 80, 81, and 85. Also suitable are fatty esters of hexitol anhydrides derived from sorbitol, such as sorbitan trioleate, sorbitan tristearate, sorbitan sesquistearate, sorbitan stearate, sorbitan palmitate, sorbitan oleate, and mixtures thereof.

The non-aqueous polar organic compound is typically employed in an amount of at least 95% by weight, preferably at least 80% by weight, more preferably at least 70% by weight, based on the weight of the composition.

Emollients

Emollients help to improve the feel and/or performance of the shaving preparations of the present invention. These emollients mimic or are compatible with a lipophilic content generally found within skin or hair. A preferred emollient is squalane. Useful emollients include, but are not limited to, fatty bodies liquid at ambient temperature, such as esters, mineral oils, animal oils, vegetable oils, synthetic oils, and silicone oils. Examples of useful esters include, but are not limited to, isononyl isononanoate, octyl palmitate, cetyl lactate, pentaerythrityl tetraoctanoate, tridecyl octanoate, tridecyl behenate, isopropyl jojobate and jojoba alcohols, butyloctyl salicylate, polyglyceryl-3 diisostearate, tridecyl trimellitate, tridecyl stearate, and neopentylglycol dicaprylate/dicaprate.

In one embodiment of the invention, the esters are chosen from isononyl isononanoate, a light ester which adds to the initial feel of the inventive composition on the skin, and pentaerythrityl tetraoctanoate. Examples of useful oils include, but are not limited to, petrolatum oil, liquid lanolin, arara oil, sesame oil, macadamia oil, jojoba oil, silicone oils such as phenyl trimethicone and dimethicone, and synthetic triglycerides such as capric caprylic triglyceride and hydrogenated cocoglycerides.

Organic oils which may be used as emollients also include esters, hydrocarbon oils, or an animal, vegetable, or mineral oil. Esters include mono-, di-, and triesters. The composition may comprise one or more esters selected from the group, or mixtures thereof. Preferably the composition contains a mixture of di- and triesters. Monoesters are defined as esters formed by the reaction of a monocarboxylic acid having the formula R-COOH, wherein R is a straight or branched chain saturated or unsaturated alkyl having 2 to 30 carbon atoms, or phenyl; and an alcohol having the formula R-OH wherein R is a straight or branched chain saturated or unsaturated alkyl having 2-30 carbon atoms, or phenyl. Both the alcohol and the acid may be substituted with one or more hydroxyl groups, and in one preferred embodiment of the invention the acid is an alpha hydroxy acid. Either one or both of the acid or alcohol may be a fatty acid or alcohol, i.e. may have from about 6 to 22 carbon atoms. Examples of monoester oils that may be used in the compositions of the invention include hexyl decyl benzoate, hexyl laurate, hexadecyl isostearate, hexyldecyl laurate, hexyldecyl octanoate, hexyldecyl oleate, hexyldecyl palmitate, hexyldecyl stearate, hexyldodecyl salicylate, hexyl isostearate, butyl acetate, butyl isostearate, butyl oleate, butyl octyl oleate, cetyl palmitate, ceyl octanoate, cetyl laurate, cetyl lactate, cetyl isononanoate, cetyl stearate, stearyl lactate, stearyl octanoate, stearyl heptanoate, stearyl stearate, and so on. It is understood that in the above nomenclature, the first term indicates the alcohol and the second term indicates the acid in the reaction, i.e. stearyl octanoate is the reaction product of stearyl alcohol and octanoic acid. Preferred is monoester which is the reaction product of an aliphatic C₂₋₈ alcohol and a C₁₄₋₂₂ fatty acid, more particularly, the reaction product of a hexyl alcohol and lauric acid, also referred to as hexyl laurate.

Suitable diesters that may be used in the compositions of the invention are the reaction product of a dicarboxylic acid and an aliphatic or aromatic alcohol. The dicarboxylic acid may contain from 2 to 30 carbon atoms, and may be in the straight or branched chain, saturated or unsaturated form. The dicarboxylic acid may be substituted with one or more hydroxyl group. The aliphatic or aromatic alcohol may also contain 2 to 30 carbon atoms, and may be in the straight or branched chain, saturated, or unsaturated form. The aliphatic or aromatic alcohol may be substituted with one or more substituents such as hydroxyl. Preferably, one or more of the acid or alcohol is a fatty acid or alcohol, i.e. contains 14-22 carbon atoms. The dicarboxylic acid may also be an alpha hydroxy acid. Examples of diester oils that may be used in the compositions of the invention include diisostearyl malate (the reaction product of isostearic alcohol and malic acid), neopentyl glycol dioctanoate (the reaction product of neopentyl glycol and 2-ethyl hexanoic acid), dibutyl sebacate (reaction product of butyl alcohol and sebacic acid), di-C₁₂₋₁₃ alkyl malate (reaction product of C₁₂₋₁₃ alcohol and malic acid), dicetearyl dimer dilinoleate (reaction product of cetearyl alcohol and adipic acid), dicetyl adipate (reaction product of cetyl alcohol and adipic acid), diisocetyl adipate (reaction product of hexadecyl alcohol and adipic acid), diisononyl adipate (reaction product of isononyl alcohol and adipic acid), diisostearyl dimer dilinoleate (reaction product of isostearyl alcohol and dilinoleic acid), disostearyl fumarate (reaction product of isostearyl alcohol and fumaric acid), and so on.

Suitable triesters comprise the reaction product of a tricarboxylic acid and an aliphatic or aromatic alcohol. As with the mono- and diesters mentioned above, the acid and alcohol contain 2 to 30 carbon atoms, and may be saturated or unsaturated, straight or branched chain, and may be substituted with one or more hydroxyl groups. Preferably, one or more of the acid or alcohol is a fatty acid or alcohol containing 14 to 22 carbon atoms. Examples of triesters include triarachidin (reaction product of glycerin and arachidic acid), tributyl citrate (reaction product of butyl alcohol and citric acid), tri C₁₂₋₁₃ alkyl citrate (reaction product of C₁₂₋₁₃ alcohol and citric acid), tricaprylin (reaction product of glycerin and caprylic acid), tricaprylyl citrate (reaction product of capryl alcohol and citric acid), tridecyl behenate (reaction product of tridecyl alcohol and behenic acid), trioctyldodecyl citrate (reaction product of octyldodecyl alcohol and citric acid), tridecyl behenate (reaction product of tridecyl alcohol and behenic acid), tridecyl cocoate (reaction product of tridecyl alcohol and coconut acid), tridecyl isononanoate (reaction product of tridecyl alcohol and isononanoate), and so on. Preferred is a triester which is the reaction product of an alpha hydroxy acid and a guerbet alcohol having 6 to 30 carbon atoms, in particular the reaction product of citric acid and octyldodecyl alcohol, referred to as trioctyldodecyl citrate.

Suitable hydrocarbon oils used in the compositions of the invention may be volatile or nonvolatile. The term volatile means that the oil has a measurable vapor pressure, or a vapor pressure of at least 2 mm. of mercury at 20°C. The term nonvolatile means that the oil has a vapor pressure of less than 2 mm. of mercury at 20°. Examples of volatile hydrocarbon oils that may be used in the compositions of the invention include various straight or branched chain paraffinic hydrocarbons having 5 to 20 carbon atoms, more preferably 8-20 carbon atoms. Suitable hydrocarbons include pentane, hexane, heptane, decane, dodecane, tetradecane, tridecane, and C₈₋₂₀ isoparaffins as disclosed in U.S. patent nos. 3,439,088 and 3,818,105, both of which are hereby incorporated by reference. Preferred volatile paraffinic hydrocarbons have a molecular weight of 70-225, preferably 160 to 190 and a boiling point range of 30 to 320, preferably 60-260°C, and a viscosity of less than 10 cs. at 25°C. Such paraffinic hydrocarbons are available from EXXON under the ISOPARS trademark, and from the Permethyl Corporation. Suitable C₁₂ isoparaffins are manufactured by Permethyl Corporation under the tradename Permethyl 99A. Another C₁₂ isoparaffin (isododecane) is distributed by Presperse under the tradename Permethyl 99A. Various C₁₆ isoparaffins commercially available, such as isohexadecane (having the tradename Permethyl R), are also suitable.

Suitable nonvolatile hydrocarbon oils include isoparaffins and olefins having greater than 20 carbon atoms. Examples of such hydrocarbon oils include C₂₄₋₂₈ olefins, C₃₀₋₄₅ olefins, C₂₀₋₄₀ isoparaffins, hydrogenated polyisobutene, mineral oil, pentahydrosqualene, squalene, squalane, and mixtures thereof Also suitable for use in the composition is lanolin oil or derivatives thereof such as hydroxylated lanolin, isobutylated lanolin oil, acetylated lanolin, acetylated lanolin alcohol, and so on.

Another useful emollient is an oleosoluble synthetic polymer whose use in cosmetic compositions is known. Representative oleosoluble synthetic polymers include polyvinylpyrrolidone/hexadecene or PVP/eicosene copolymers, such as products sold by GAF Corp. under the tradenames GANEX V-216 and GANEX V-220. Other useful skin conditioning emollients are listed in the International Cosmetic Ingredient Dictionary and Handbook, 7th Edition, Vol. 2, pp. 1656-1661 (1997).

Another suitable type of emollient is a silicone, such as a polysiloxane compound. Examples of suitable polysiloxanes include, but are not limited to, phenyl-functional polymethylsiloxane compounds (e.g., Dow Coming 556 Cosmetic-Grade Fluid) and cetyl or stearyl functionalized dimethicones such as Dow 2502, General Electric SF1632 and Dow 2503 polysiloxane fluids. In addition to such substitution with phenyl-functional or alkyl groups, effective substitution may be made with amino, carboxyl, hydroxyl, ether, polyether, aldehyde, ketone, amide, ester, and thiol groups. Phenyl, amino, alkyl, carboxyl, and hydroxyl groups are preferred, with phenyl functional groups being most preferred.

When used, the amount of emollient should range from between 0.01 to 20% by weight, preferably from 1 to 10% by weight, and more preferably from 3 to 5% by weight, based on the weight of the composition.

Thickeners

The present compositions may also contain a thickener. Examples of thickeners usable according to the invention include, but are not limited to:
water-soluble cellulosic thickeners such as hydroxyethylcellulose, methylcellulose, and hydroxypropylcellulose. A commercially available example includes, but is not limited to, CELLOSIZE QP 44001H, from Amercol;
guar gum, such as those sold under the name VIDOGUM GH 175, from Unipectine, and JAGUAR C, from Meyhall;
quaternized guar gum, such as JAGUAR C-13-S, from Meyhall;
non-ionic guar gums containing C₁-C₆ hydroxyalkyl groups. Examples include, but are not limited to, hydroxymethyl, hydroxyethyl, hydroxypropyl and hydroxybutyl. Commercially available examples include, but are not limited to, JAGUAR HP8, JAGUAR HP60 and JAGUAR HP120, JAGUAR DC 293, and JAGUAR HP 105, from Meyhall, and GALACTASOL 4H4FD2, from Aqualon;
xanthan, carob, scleroglucan, gellan, rhamsan, and karaya gums;
alginates, maltodextrin, starch and derivatives thereof, hyaluronic acid and salts thereof;
clays, such as montmorillonites, hectorites and laponites;
crosslinked polyacrylic acids such as CARBOPOL, from Goodrich
glyceryl poly(meth)acrylate polymers, such as those sold under the names HISPAGEL or LUBRAGEL by the companies Hispano Quimica or Guardian
polyvinylpyrrolidone;
polyvinyl alcohol;
crosslinked polymers and copolymers of acrylamide, such as those sold under the names "PAS 5161" or BOZEPOL C, from Hoechst, and SEPIGEL 305, from Seppic
crosslinked methacryloyloxyethyltrimethylammonium chloride homopolymers, such as those sold under the name SALCARE SC95, from Allied Colloid
aggregating polymers, including aggregating polyurethanes; and
highly crosslinked polyacrylamidomethylpropane sulfonic acid partially neutralized with ammonia such as, for example, ammonium polyacryloyldimethyltaurate, commercially available as Hostacerin AMPS from Clariant.

In the composition according to the invention, the thickener may be present in an amount ranging from 0.01% to 10% by weight, preferably from 0.3 to 6% by weight, and more preferably from 0.3 to 0.5% by weight, based on the weight of the composition.

Fragrances

Fragrances, as used in this application, are made by selecting and combining materials published in Allured Flavor and Fragrance Materials (Allured Publishing Co., Carol Stream, Ill., 1997 ed.). These compounds include various esters, aldehydes, alcohols, ketones, terpenes, ethers, acetals, nitrites, essential oils, heterocyclic nitrogen-containing compounds or sulfur-containing compounds. Examples of phenolic fragrances appropriate for use in the present invention include amyl salicylate, cavacrol, dihydroeugenol, eugenol, hexyl eugenol, hexyl salicylate, isoeugenol, methyl eugenol, methyl isoeugenol, methyl salicylate, tert butyl cresol, thymol, and vanillin. Examples of non-aromatic terpenoid compounds include cedrene, cineole, citral, citronellal, citronellol, cymene, paradihydrolinalool, dihydromyrcenol (DH myrcenol), farnesol, geraniol, hexyl cinnamaldehyde, hydroxycitronallol, hydroxycitronellal, isocitral, limonene, linalool, longifolene, menthol, nerol, nerolidiol, phellendrene, terpinene, terpinenol, and tetrahydromyrcenol (TH myrcenol).

The phenolic compounds and non-aromatic terpenoids may be added in an isolated form. Alternatively or additionally, essential oils containing the phenolic compounds and/or the non-aromatic terpenoids as major constituents may be added, with the final concentrations of the phenolic compounds and the non-aromatic terpenoids being within the range of the invention. The term "major constituent" refers to those essential oils having phenolic compounds or non-aromatic terpenoids, wherein the phenolic compounds or non-aromatic terpenoids constitute more than 50% by weight of the composition of the essential oil. It is well-known in the art that such essential oils may also contain lesser amounts of the other constituent, i.e., essential oils containing phenolic compounds often contain lesser amounts of non-aromatic compounds, and essential oils containing non-aromatic compounds often contain lesser amounts of phenolic compounds. Essential oils including phenolic compounds as the major constituent include, for example, anise oil, bay oil terpineless, clove bud, clove leaf, clove oil, clove stem, origanum oil, Peru balsam, pimento oil, and thyme oil. Essential oils including non-aromatic terpenoids as the major constituent include, for example, buchu oil, caraway oil, carrot seed, cedar leaf, citronella oil, citrus oil, copaiba oil, geranium oil, gergamot, lavender oil, mint oil, orange oil, parsley oil, patchouly oil, pine oil, rosemary oil, sage oil, tagette oil, and ylang ylang.

Fragrances may be employed in an amount of from 0.01 to 5% by weight, preferably from 0.1 to 4% by weight, and more preferably from 0.1 to 3% by weight, based on the weight of the composition.

In the event that it is desirable to include some water in the compositions of the present invention, the water may be employed in an amount of up to 20% by weight, preferably up to 10% by weight, and more preferably up to 5% by weight, based on the weight of the composition.

To improve trackability (the ability of a consumer to see which areas have been shaved and which have not), it may be desirable to add a pigment. A preferred pigment is titanium dioxide (TiO₂). The precise amount of titanium dioxide to be used will depend upon, and be determined by, one of ordinary skill in the art. It should be noted that any equivalent pigments may be used as desired.

The composition of our invention may also include plastic particles, such as beads, in an amount of from 0.1 to 20% by weight, preferably from 1 to 10% by weight, and more preferably from 2 to 5% by weight, based on the weight of the composition. The addition of plastic particles helps loosen dead skin for removal during shaving. Preferred plastic particles include oxidized polyethylene beads, which can be a mixture of Acumist A45 beads, average particle size of about 45µm (from Allied Signal of Morristown, N.J.); 316A beads, average particle size of about 141µm (from Allied Signal); and 9F beads, average particle size of about 110µm (from Allied Signal).

Other common shaving preparation and cosmetic additives may be used which include, but are not limited to, menthol, camphor, methyl salicylate, vitamin E acetate USP, aloe vera powder, fragrances, colorants, and preservatives, such as methylparaben, propylparaben, and GERMABEN II-E (diazolidinyl urea and parabens preservative).

In a preferred form, the shaving preparation of the present invention is essentially free of soap. By essentially free of soap is meant less than 10% by weight, preferably less than 7% by weight, and more preferably less than 5% by weight, based on the weight of the composition.

According to one embodiment of the present invention, there is provided a shaving preparation containing: (a) from 50 to 95% by weight, preferably from 60 to 85% by weight, and more preferably from 70 to 80% by weight, of at least one non-aqueous polar organic compound, preferably propylene glycol; (b) from 0.01 to 20% by weight, preferably from 1 to 10% by weight, and more preferably from 3 to 5% by weight, of at least one emollient, preferably squalane; (c) from 0.01 to 10% by weight, preferably from 0.3 to 6% by weight, and more preferably from 0.3 to 0.5% by weight, of at least one thickener; (d) from 0.01 to 5% by weight, preferably from 0.1 to 4% by weight, and more preferably from 0.1 to 3% by weight, of at least one fragrance, preferably an essential oil; and (e) optionally, from 1 to 20% by weight, preferably from 2 to 15% by weight, and more preferably from 5 to 10% by weight, of at least one surfactant, preferably a nonionic surfactant, all weights being based on the weight of the composition.

The present invention is also directed to a process for shaving a surface, such as human skin having hair projecting therefrom. The process involves the steps of: (a) providing a surface to be shaved; (b) providing a composition containing: (i) at least one non-aqueous polar organic compound having at least two hydroxyl groups; (ii) optionally, at least one emollient; (iii) optionally, at least one thickening agent; (iv) optionally, at least one fragrance; and (v) optionally, water; (c) optionally, wetting the surface to be shaved; (d) applying the composition onto the surface to be shaved in order to form a treated surface; and (e) shaving the treated surface.

The instrument used to perform the shaving step may be any conventional shaving device such as a razor or electric shaver.

The present invention will be better understood from the examples which follow, all of which are intended for illustrative purposes only and are not meant to unduly limit the scope of the invention in any way.

EXAMPLES

Example 1

| Ingredient | % |
|---|---|
| Glyceryl oleate/coco glucoside | 1.00 |
| glycerin | 10.00 |
| Methyl gluceth-20 | 10.00 |
| Propylene glycol | 45.00 |
| PEG-6 | 20.42 |
| Glycereth-26 | 10.00 |
| AMPS | 0.30 |
| Dimethicone PEG-7 phosphate | 3.00 |
| Mentha viridis leaf | 0.11 |
| Eucalyptus globulus leaf oil | 0.03 |
| Cedrus atlantica bark oil | 0.07 |
| Rosmarinus officinalis leaf oil | 0.07 |
| Total | 100.00 |

Example 2

| Ingredient | % |
|---|---|
| Glyceryl oleate/coco glucoside | 5.00 |
| glycerin | 9.20 |
| Methyl gluceth-20 | 9.20 |
| Propylene glycol | 45.00 |
| PEG-6 | 20.00 |
| Glycereth-26 | 9.02 |
| AMPS | 0.30 |
| Dimethicone PEG-7 phosphate | 2.00 |
| Mentha viridis leaf | 0.11 |
| Eucalyptus globulus leaf oil | 0.03 |
| Cedrus atlantica bark oil | 0.07 |
| Rosmarinus officinalis leaf oil | 0.07 |
| Total | 100.00 |

Example 3

| Ingredient | % |
|---|---|
| Glyceryl oleate/coco glucoside | 1.00 |
| glycerin | 10.00 |
| Methyl gluceth-20 | 9.40 |
| Propylene glycol | 45.00 |
| PEG-6 | 21.00 |
| Glycereth-26 | 11.02 |
| AMPS | 0.30 |
| Dimethicone PEG-7 phosphate | 2.00 |
| Mentha viridis leaf | 0.11 |
| Eucalyptus globulus leaf oil | 0.03 |
| Cedrus atlantica bark oil | 0.07 |
| Rosmarinus officinalis leaf oil | 0.07 |
| Total | 100.00 |

Example 4

| Ingredient | % |
|---|---|
| Glyceryl oleate/coco glucoside | 2.00 |
| glycerin | 10.00 |
| Methyl gluceth-20 | 9.40 |
| Propylene glycol | 45.00 |
| PEG-6 | 21.00 |
| Glycereth-26 | 10.02 |
| Ammonium polyacrylodimethyl taurate | 0.30 |
| Dimethicone PEG-7 phosphate | 2.00 |
| Mentha viridis leaf | 0.11 |
| Eucalyptus globulus leaf oil | 0.03 |
| Cedrus atlantica bark oil | 0.07 |
| Rosmarinus officinalis leaf oil | 0.07 |
| Total | 100.00 |

The shaving preparations exemplified above exhibited excellent rinsability and lubricity.

## Claims

1. A process for shaving a surface having hair projecting therefrom comprising:
(a) providing a surface to be shaved;
(b) providing a composition containing:
a. at least one non-aqueous polar organic compound having at least two hydroxyl groups;
b. optionally, at least one emollient;
c. optionally, at least one thickening agent;
d. optionally, at least one fragrance; and
e. optionally, water;
(c) optionally, wetting the surface to be shaved;
(d) applying the composition onto the surface to be shaved in order to form a treated surface; and
(e) shaving the treated surface.

2. The process of claim 1 wherein the at least one non-aqueous polar organic compound having at least two hydroxyl groups is propylene glycol.

3. The process of claim 1 wherein the at least one non-aqueous polar organic compound having at least two hydroxyl groups is present in the composition in an amount of at least about 50% by weight, based on the weight of the composition.

4. The process of claim 1 wherein the at least one non-aqueous polar organic compound having at least two hydroxyl groups is present in the composition in an amount of at least about 70% by weight, based on the weight of the composition.

5. The process of claim 1 wherein the at least one emollient is squalane.

6. The process of claim 1 wherein the at least one emollient is present in the composition in an amount of from about 0.01 to about 20% by weight, based on the weight of the composition.

7. The process of claim 1 wherein the at least one emollient is present in the composition in an amount of from about 3 to about 5% by weight, based on the weight of the composition.

8. The process of claim 1 wherein the at least one thickening agent is a highly crosslinked polyacrylamidomethylpropane sulfonic acid partially neutralized with ammonia.

9. The process of claim 1 wherein the at least one thickening agent is present in the composition in an amount of from about 0.01 to about 10% by weight, based on the weight of the composition.

10. The process of claim 1 wherein the at least one thickening agent is present in the composition in an amount of from about 0.3 to about 0.5% by weight, based on the weight of the composition.

11. The process of claim 1 wherein the at least one fragrance is an essential oil.

12. The process of claim 1 wherein the at least one fragrance is present in the composition in an amount of from about 0.01 to about 5% by weight, based on the weight of the composition.

13. The process of claim 1 wherein the at least one fragrance is present in the composition in an amount of from about 0.1 to about 3% by weight, based on the weight of the composition.

14. The process of claim 1 wherein the water is present in the composition in an amount of up to about 20% by weight, based on the weight of the composition.

15. A composition comprising:
(a) at least 50% by weight of at least one non-aqueous polar organic compound having at least two hydroxyl groups;
(b) from 0.01 to 20% by weight of at least one emollient;
(c) from 0.01 to 10% by weight of at least one thickening agent;
(d) from 0.01 to 5% by weight of at least one fragrance; and
(e) optionally, up to 20% by weight, water, all weights being based on the total weight of the composition, and wherein the composition is used for shaving a surface having hair projecting therefrom.

16. The composition of claim 15 wherein the at least one non-aqueous polar organic compound having at least two hydroxyl groups is propylene glycol.

17. The process of claim 1 wherein the at least one non-aqueous polar organic compound having at least two hydroxyl groups is present in the composition in an amount of at least about 50% by weight, based on the weight of the composition.

18. The process of claim 1 wherein the at least one non-aqueous polar organic compound having at least two hydroxyl groups is present in the composition in an amount of at least about 70% by weight, based on the weight of the composition.

19. The process of claim 1 wherein the at least one emollient is present in the composition in an amount of from about 0.01 to about 20% by weight, based on the weight of the composition.

20. The process of claim 1 wherein the at least one emollient is present in the composition in an amount of from about 3 to about 5% by weight, based on the weight of the composition.

21. The process of claim 1 wherein the at least one thickening agent is present in the composition in an amount of from about 0.01 to about 10% by weight, based on the weight of the composition.

22. The process of claim 1 wherein the at least one thickening agent is present in the composition in an amount of from about 0.3 to about 0.5% by weight, based on the weight of the composition.

23. The process of claim 1 wherein the at least one fragrance is an essential oil.

24. The process of claim 1 wherein the at least one fragrance is present in the composition in an amount of from about 0.01 to about 5% by weight, based on the weight of the composition.

25. The process of claim 1 wherein the at least one fragrance is present in the composition in an amount of from about 0.1 to about 3% by weight, based on the weight of the composition.
